# EUROPEAN PATENT APPLICATION

(11) **EP 1 334 706 A1**
(43) Date of publication of application: **13.08.2003**
(21) Application number: 01949943.3
(22) Date of filing: 11.07.2001
(51) Int. Cl.: A61F 5/445

(54) **POUCH FOR STOMA AND METHOD FOR TREATING**

(30) Priority: 18.11.2000 JP 2000391616
(71) Applicant: Sakamoto, Atsunobo, Inba-gun, Chiba 270-1423 (JP)
(72) Inventor: Sakamoto, Atsunobo, Inba-gun, Chiba 270-1423 (JP)
(74) Representative: Thomson, Paul Anthony
(86) International application number: JP0106018
(87) International publication number: WO02039936

(57) **Abstract**

A method for treating the excrement in a pouch, characterized in that it utilizes the fact that the pouch is generally made of a thermoplastic resin such as polyethylene, and comprises gathering the excrement in the bottom portion of the pouch, heat-sealing an appropriate position between the bottom portion containing the excrement and an upper portion by using a heat sealer, cutting the pouch at an approximate middle of the heat-sealed part into two sealed pouches, to thereby remove the excrement with no odor going out. Also are disclosed a pouch which is so improved as to facilitate employing the above method, and an impulse heat sealer which is so improved as to be lightweight, be washable with ease, and be usable with weak power.

## Description

### FIELD OF THE INVENTION

The present invention relates generally to improved devices and methods for managing body wastes by ostomates, i.e., persons who have had a colostomy, ileostomy, urostomy, or similar operation whereby bodily waste is diverted for discharge through a surgically created opening, or stoma, and collected in a pouch.

### BACKGROUND OF THE INVENTION

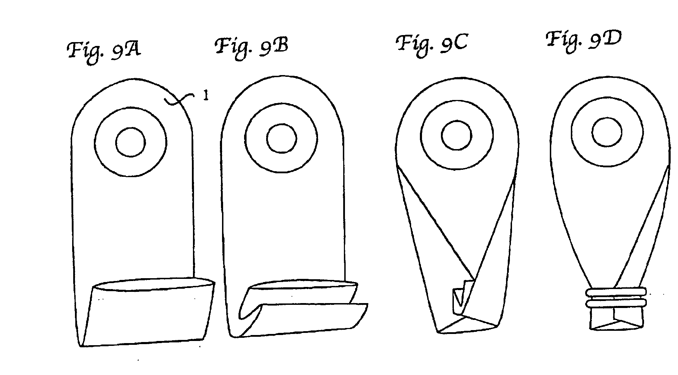

For many years, pouches with open bottoms have been used to collect the discharged waste products of ostomates. After a substantial amount of waste has passed into the pouch, the bottom is opened and the waste is removed. Thereafter, the bottom of the pouch must be washed. To prevent leakage of urine and/or feces, the open bottom of a pouch is usually sealed by clips or by folding into layers and cinching with rubber bands as shown in Fig.9A - 9D. Each of these methods of closing the pouch has undesirable disadvantages. The use of clips is simple, but leakage commonly occurs. Likewise, sealing by rubber bands is time consuming and complex because the pouch must be intricately folded.

Pouches with closed bottoms are available for one-day or relatively short-term usage but must be replaced daily or at similar, frequent intervals. Replacement of pouches requires significant time and effort because the stoma and peristomal skin must be cleansed prior to affixing a new pouch. Due to the increased frequency of pouch-changing, the monetary cost increases, and locating appropriate places for performing the procedure becomes more difficult.

Needless to say, strong odor is discharged during disposal of the stored waste, which is unpleasant for the ostomate, or those who care for the ostomate. This fact limits and restrains ostomates and tends to prevent them from going out, traveling, or enjoying any outdoor or athletic activities. In the case of urine, although the odor is less strong, the same problem often exists.

### BRIEF SUMMARY OF THE INVENTION

A method for the disposal, with no odor escaping, of human waste products stored in a pouch is disclosed using heat-seal and heat-cut methods applied by an impulse sealer to a pouch generally made of thermoplastic such as polyethylene resin. Also disclosed is a method of ensuring the integrity of the heat-cut edge by re-sealing the pouch to eliminate possible leakage or odor.

To apply these methods, two types of pouch are disclosed. One is an elongate pouch, longer than a standard ostomy pouch, while the other is a pouch suitable for extension by the addition of lengths of another pouch or other material. Below the adhesive region surrounding the stoma, ink or other suitable media is printed across the width of one interior wall of the latter pouch to prevent the walls from being fused together.

An improved impulse heat sealer also is disclosed, in which an impulse heat sealer, traditionally used in manufacturing factories, is suitably modified so as to be lightweight, washable, and usable requiring reduced gripping power. Viewed in cross-section through its center, the heating wire of the heat sealer exhibits a convex shape.

### BRIEF DESCRIPTION OF DRAWINGS

Fig.1A illustrates in plane view the methods of this invention.
Fig.1B illustrates in plane view the methods of this invention.
Fig.1C illustrates in plane view the methods of this invention.
Fig.2 is the cross section of a sealer and a pouch.
Fig.3 is an enlarged, cross-sectional view of the sealed pouch.
Fig.4A is an enlarged, cross-sectional view of a heating element.
Fig.4B is an enlarged, cross-sectional view of a heating element.
Fig.4C is an enlarged, cross-sectional view of a heating element.
Fig.5 illustrates in plane view the method of sealing the cut edge of a pouch.
Fig.6 illustrates in plane view the method of extending a pouch.
Fig.7 is a side view of the impulse heat sealer of this invention.
Fig.8 is a graph showing the results of comparative experiments for the treatment of body waste.
Fig.9A illustrates in plane view a conventional method for the treatment of body waste.
Fig.9B illustrates in plane view a conventional method for the treatment of body waste.
Fig.9C illustrates in plane view a conventional method for the treatment of body waste.
Fig.9D illustrates in plane view a conventional method for the treatment of body waste.

### DETAILED DESCRIPTION OF THE PREFERRED EMBODIMENT

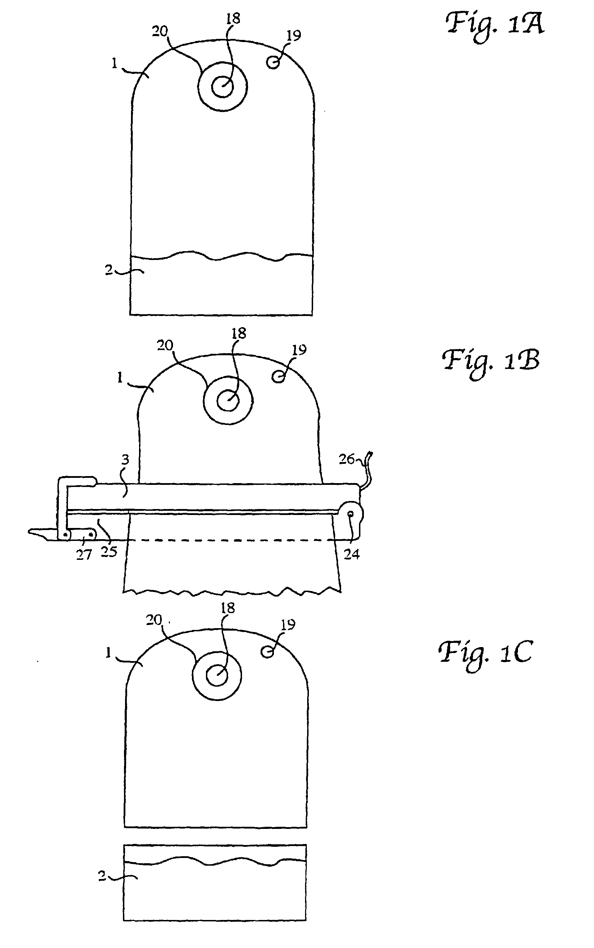

In Fig. 1A, human waste product 2 has collected in the bottom portion of pouch 1. Thereafter, an impulse heat sealer 3 sandwiches a portion of pouch 1 slightly above the collected waste and applies pressure while an approximate one (1) second pulse of electrical current creates heat for sealing pouch 1 along a transverse seam or seal line. Impulse heat sealer 3 is held in place for approximately an additional two (2) seconds to allow the seal to cool. By pulling firmly above and below the transverse seal line, the pouch 1 may advantageously be divided into two separate, sealed pouches substantially as depicted in Fig. 1C. The sealed pouch with the waste 2 then may be appropriately discarded. While the portion of pouch 1 still attached to the ostomate's torso is shortened, it contains no waste and is still usable without any further modification or need for cleaning. When the foregoing method is employed, no odor escapes from the pouch because waste 2 is not exposed to the outside air.

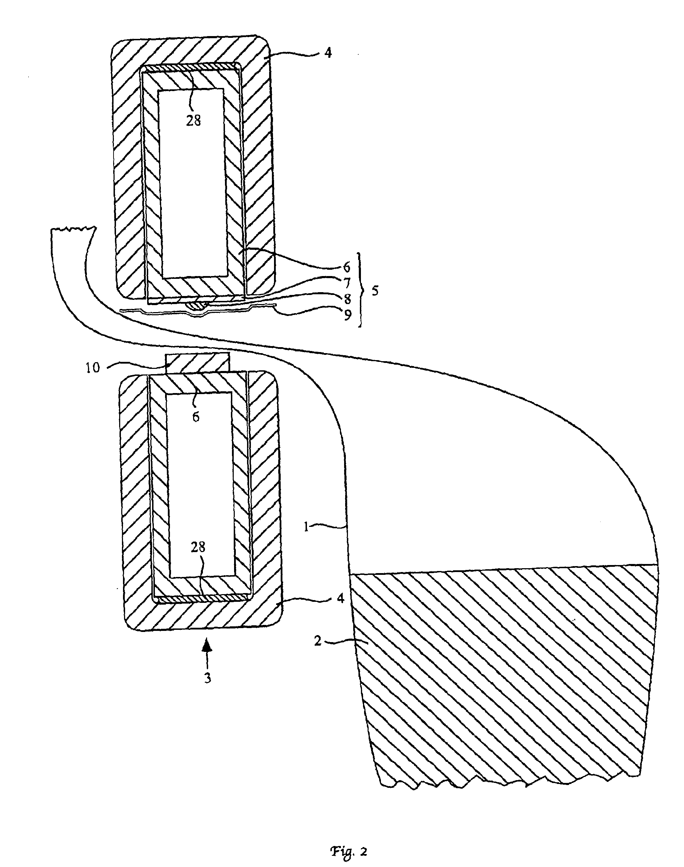

More details are shown in Fig.2, which shows a cross-section of impulse heat sealer 3. The impulse heat sealer 3 is comprised of two frames 4, into one of which a heating element 5 is mounted. The heating element 5 is comprised of a sheet of insulating fabric, such as glass tape 7, sandwiched between an aluminum bar 6 and a Nichrome heating wire 8 which exhibits a convex cross-sectional shape and is coated with an adhesive tape of tetrafluoroethylene 9. Likewise, an additional aluminum bar 6 is mounted in the other frame 4 with silicon rubber 10 affixed to the surface facing the heating element 5.

To obviate waste from the region to be sealed, pouch 1 is squeezed or pressed/pinched together. Then, pouch 1 is squeezed and pinched between heating element 5 and silicon rubber 10 of impulse heat sealer 3.

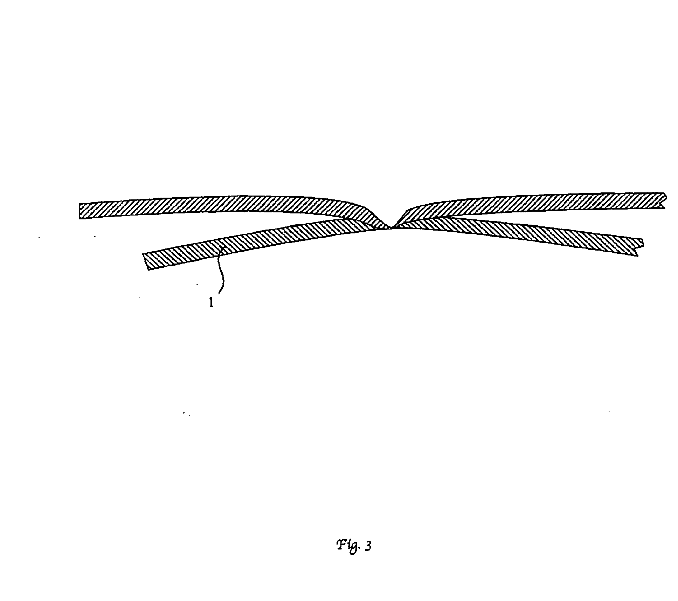

When the proper amount of current flows through the electric circuit of the sealer, heating element 5 generates heat and heat-seals pouch 1 within one (1) second, melting and welding the two films comprising the opposed walls of the pouch into one, thinner film as shown in Fig. 3. After Impulse heat sealer 3 is held in place for approximately an additional two (2) seconds to allow the seal to cool, pulling firmly above and below the seal, or transversely cutting the central portion of the seal, divides pouch 1 into two separate, sealed pouches. If the electric current is substantially strong, the pouch may be heat-sealed and heat-cut simultaneously along the transverse seam or seal line.

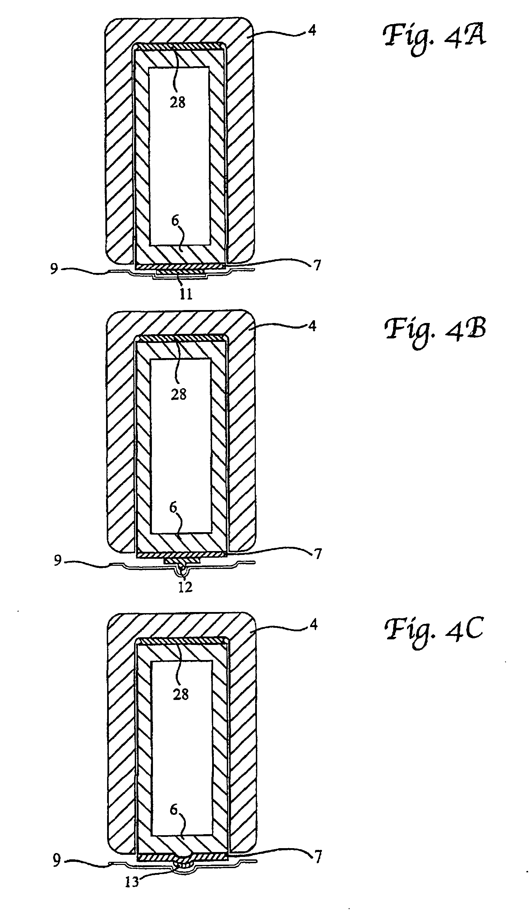

As shown in Fig. 4A, heating element 5 may employ a flat wire 11 of an approximate 2 to 5 mm width, defining the transverse sealed region to be cut thereafter. Additionally, as shown in Fig. 4B, heating element 5 may employ a flat Nichrome wire of the approximate height and width of 0.1 mm and 2 mm, respectively, with a round Nichrome wire of the approximate diameter of 0.3 mm welded on top thereof, resulting in a heating wire 12 capable of heat-sealing and heat-cutting simultaneously. However, because the size and shape of the above-mentioned heating wires may not be appropriate to sufficiently obviate waste products from the region to be sealed, the integrity of the seal may be compromised, allowing odor and waste possibly to escape.

The shape of heating wire 8 as shown in Fig. 2, or an aluminum bar with a protrusion 6 and ribbon wire 13 atop thereof as shown in Fig. 4C, produce the most desirable results. With such arrangements, the wire surface exhibits a convex cross-sectional shape, and, when pressure is applied, any waste product between films of pouch 1 is more effectively obviated. Moreover, these heating wires produce a transverse cut edge which is smooth and regular in shape.

However, the shape of the heating wire, e.g., 8, 11, 12, and 13, along with the amount of pressure applied to the ostomy pouch may not be sufficient to completely obviate waste products from the region to be sealed, especially when feces or other waste are fibrous. In this case, the seal may be contaminated and prone to leakage. The usage of adhesive tape is not sufficient to seal the cut edge due to this surface's irregular shape.

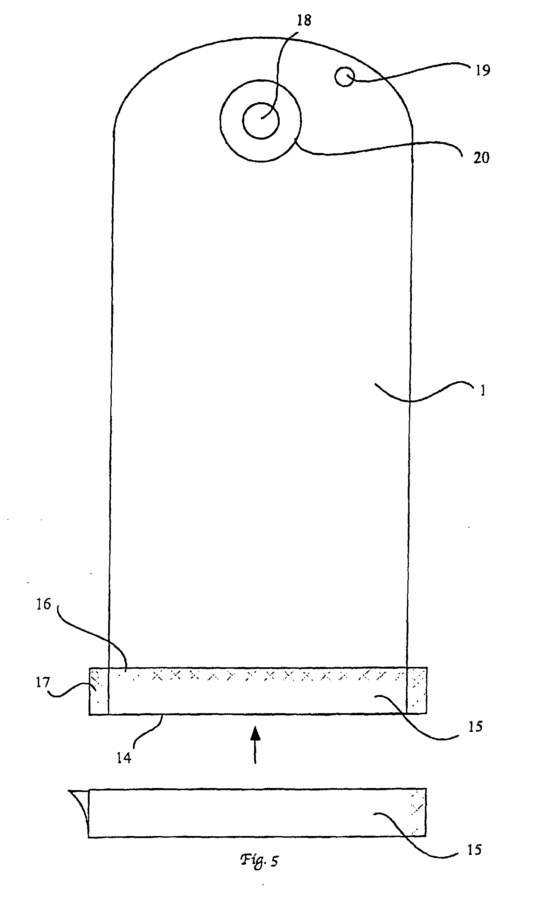

The solution, presented in Fig. 5, is to seal the cut edge 14 of pouch 1 with a second pouch or a thermoplastic sheet 15, which is of a transverse extent greater than that of pouch 1. The second pouch or the sheet 15 is bi-folded and one of the short sides is heat-sealed, producing two closed edges. The second pouch or the sheet 15 is then mated to edge 14 of pouch 1 such that the longer, open edge of the second pouch or the sheet 15 overlaps with and encompasses edge 14. A seal 16 is created by subsequently heat-sealing the long or transverse edge of the second pouch or the sheet 15. Pouch 1 is re-sealed and completely closed by creating an additional seal 17 at the short, open edge of the second pouch or the sheet 15. This method may be performed beginning with three edges of the second pouch or the sheet 15 closed, but executing the method becomes more difficult. The preferred method for sealing edge 14 is by the second pouch or the sheet 15 with two sides closed, wherein the longer edge is either bi-folded or, alternately, heat-sealed.

Since the treatment of waste under the above procedures prevents gas and odor from escaping, any residual gas inside pouch 1 must be extracted. The gas is preferably extracted through a sealable opening 19 in the upper part of pouch 1. The opening 19 may be sealed with either a lid or a typical stopper, and should be placed above the stoma opening 18 as shown in Fig.1A-C, Fig.5, and Fig.6. Opening 19 may be used to attach pouch 1 to a deodorizing machine.

The length of the pouch for the use of this invention requires at least five (5) cm each time when pouch 1 is sealed and the lower portion is removed, which may be done approximately three times per day. A minimum of forty-five (45) cm is required for three days' usage. Including the adhesive region, this length increases to as much as sixty (60) cm, which may be difficult to manage. Therefore, as pouch 1 becomes short due to continued usage, it is desirable to lengthen it by adding a second thermoplastic pouch.

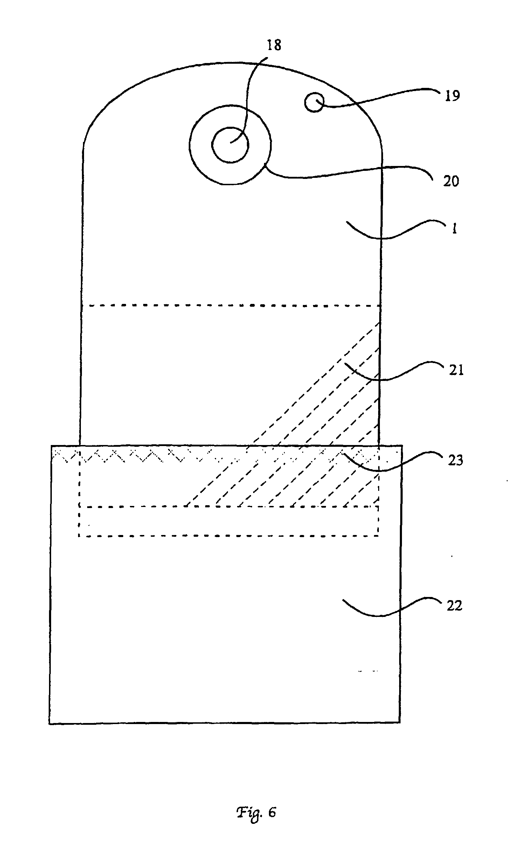

In order to lengthen pouch 1, as shown in Fig. 6, a thin sheet of polyester film 21 of substantially identical transverse extent is fully inserted into the open bottom of pouch 1, below planer adhesive region 20, so that the lower edges of pouch 1 and polyester film 21 are approximately aligned. Thereafter, second thermoplastic pouch 22 is slid over the exterior of pouch 1 so that it overlaps with polyester film 21. Upon heat-sealing, heat is transmitted through all five layers of film; however, polyester film 21, since it is thin but not thermoplastic, conducts the heat without melting. As a result, pouch 1 and second pouch 22 are welded together, creating a single, lengthened, leak-resistant pouch. Polyester film 21, which prevents the interior opposed walls of the pouch from fusing together, is removed. Subsequently, the open bottom of second pouch 22 is sealed.

Alternately, for lengthening pouch 1, coating with ink or other suitable media is as effective as polyester film 21 in preventing the fusing of the interior, opposed walls. A suitable distance below planar adhesive region 20, the entire width of at least one interior wall of pouch 1 is coated, providing approximately equivalent coverage to the same area as polyester film 21 in Fig.6. In this manner, pouch 1 is capable of being lengthened as needed, and, if the region above seal 23 is cut and removed, pouch 1 may be lengthened multiple times.

While conventional radiant heat sealers, i.e., sealers, which must be pre-heated to a suitable temperature, are available for the use of this invention, impulse heat sealers, which reach their working temperature within approximately one (1) second by generating a large pulse of electrical current, are more convenient. However, conventional impulse heat sealers are designed for industrial use, and typically not suitable for portable use, even as desktop models, because of the heavy weight of built-in transformers.

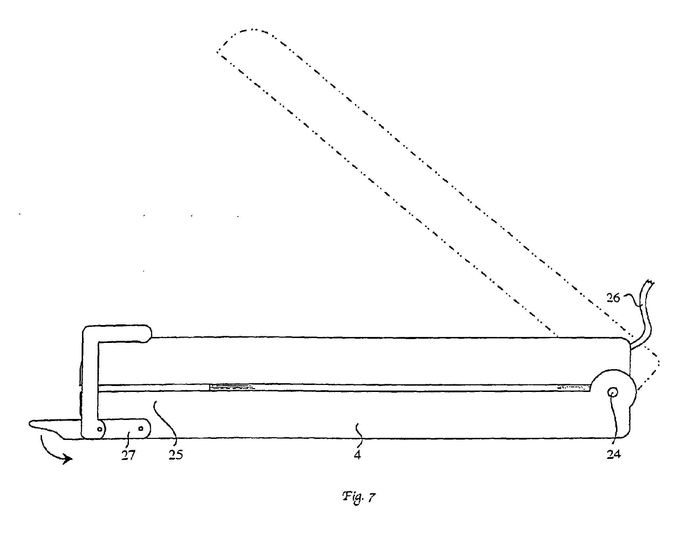

Thus, an improved, portable impulse heat sealer is shown in Fig.7. Frames 4 are constructed of flexible, heat-resistant plastic, such as polycarbonate, which permits sterilization in boiling water. Two frames 4 of substantial length, situated in a confronting position, are mutually coupled at one end and allowed to rotate around a fulcrum 24. The opposite ends of frames 4 are respective handles 25 for providing leverage and pressure by hand. An aluminum bar with a heating element is mounted in one frame, and the other aluminum bar with silicon rubber is mounted in the other frame.

Furthermore, the structure is improved by mounting a power circuit, such as a transformer and a switch, outside of the frames so that the frames are washable and lightweight, enabling the sealer to be hand-held, which, in turn, allows the sealer to be easily handled in close proximity to one's body. In Fig. 7, numeral 26 is a power cord.

Moreover, mounting a lock or clamp 27 at the ends of the handles substantially as shown reduces the required force and produces firm, even pressure throughout the length of the sealer. Hence, people with reduced strength can easily operate the sealer. By varying the pressure applied through the handles, waste may be cleared from the area to be sealed, assuring a clean seal.

The sealer requires an electric potential of 100 - 110 V that is transformed to 12 V through the power circuit. In this case, the time to seal a fifteen (15) cm wide pouch increases to more than 2 seconds. However, in the above case, the sealer may be powered by standard automobile voltage. As a result, a transformer is not necessary, and the weight of the improved impulse heat sealer is further decreased relative to conventional impulse heat sealers. At the same time, locations for use become much more widely available.

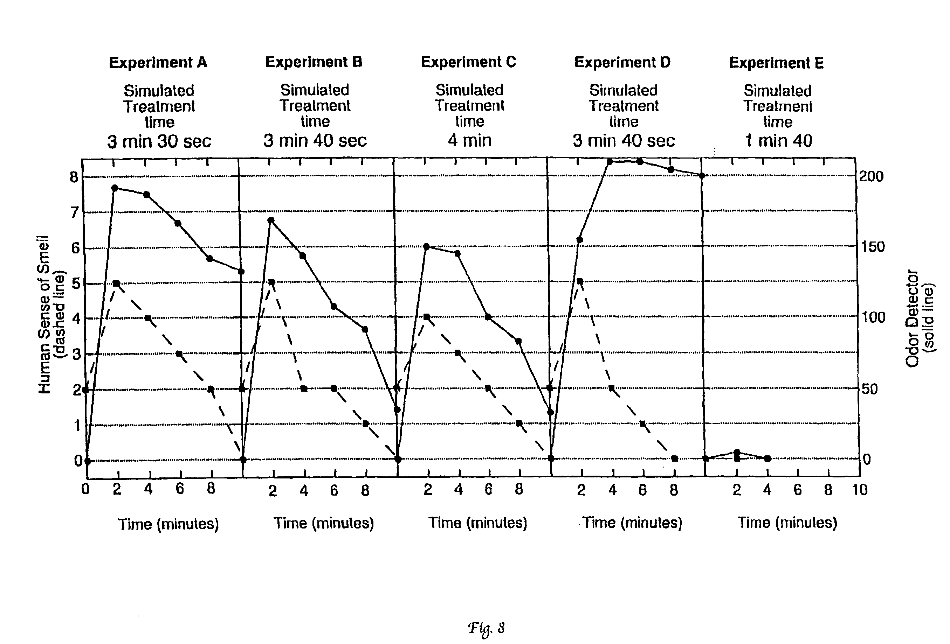

The graphs of Fig. 8 illustrate the results of five comparative experiments between the waste treatment of this invention and conventional waste treatments. Procedures of each experiment are as follows:
A. Waste is put in a paper cup. The paper cup is left on the floor until the bottom of the ostomy pouch is closed with a rubber band. The cup is then put in a plastic bag, and the bag is removed from the room.
B. Waste is put in a paper cup. Immediately, the cup is put in a plastic bag and the opening of the bag is tied. The bottom of the ostomy pouch is closed with a rubber band.
C. 6 drops of deodorant are placed in the ostomy pouch in advance. The product name of the deodorant is "Vanish." The rest of procedure is the same as B.
D. Deodorant is sprayed in the room after the procedure of B is completed.
E. The procedure is done by the impulse sealer of this invention.

The human sense of smell and an odor detector were used to evaluate the strength of the odor released. The evaluation by human sense of smell is the average of the results of three healthy adult women shown as a dotted line with values ranging from 0 to 5. The name of the odor detector is Odor Sensor XP-329 (Alcare Corporation) and the result is shown as a solid line with values ranging from 0 to 200. The waste used was 50 g of feces from a healthy three-year-old child. The evaluation was performed 50 cm from the site of the simulated treatment of waste. The temperature of the room was 25°C, and the relative humidity was 60%. Each experiment was performed with the window closed during the simulated treatment time. Simulated treatment time is the time required for a person to open an empty ostomy pouch, simulate the treatment of waste, put on clothes, close the bottom of the ostomy pouch with a rubber band, and leave the room. The window was opened immediately after each simulated treatment finished.

The 6 levels (0 - 5) of human sense of smell, as relating to the experiments, are shown below:
Level 0: No smell
Level 1: Faint small
Level 2: Faint but identifiable smell
Level 3: Easily noticeable smell
Level 4: Moderately strong smell
Level 5: Strong smell

It is obvious from these experiments that it is impossible to eliminate the strong odor by conventional methods, such as Experiments A through D. On the other hand, by using the impulse sealer in accordance with the present invention, as in Experiment E, the treatment of waste is easily accomplished with no odor escaping. Moreover, the procedure employing the impulse sealer of the invention required less than half the time of conventional procedures. In the present sealing method, the impulse sealer never comes in contact with the person's body.

### PROFITABILITY AND INDUSTRIAL APPLICABILITY

This invention allows easy treatment of waste products exited through a stoma with pouches or the like. Under the methods of this invention, the site of treatment is kept clean and no leakage occurs after treatment, even if the ostomate or the ostomate's caregiver is elderly or disabled. Because no waste or smell escapes, ostomates will find more suitable places for treatment of any waste, as long as 12 V or 100 - 110 V electric power is available, and they may feel less apprehensive about asking other people for assistance.

Employing the methods and impulse heat sealer of this invention, users are able to attach extensions to lengthen pouches. Also, users may still utilize conventional pouches. Pouches may initially be as short as possible and lengthened as necessary.

This invention may allow ostomates with a limited lifestyle, or those who are nervous about meeting people, to enjoy a more normal way of life, freeing them from much anxiety.

This invention is more cost effective when compared to conventional methods, which require both hot water and electricity for washing and drying after each treatment, while the methods of this invention only require bags for extension and a small amount of electricity.

Traditional impulse heat sealers, typically only convenient for industrial use, have been improved for the purpose of ostomy pouch treatment. This resulted from the improvement of the composition of the heat sealer to provide portability and usability for people with reduced strength.

## Claims

1. A method for treatment of human waste in a pouch, utilizing the facts that said pouch is generally of thermoplastic materials such as polyethylene, comprising the steps of:
concentrating collected waste in the bottom portion of said pouch, and
heat-sealing an appropriate position between the bottom portion containing said waste and an upper portion by using an impulse heat sealer, and
simultaneously or subsequently cutting said pouch at approximately the middle of the heat-sealed part, forming two sealed pouches, and
removing the pouch containing said waste,
whereby said waste is collected for disposal in a sealed pouch without odor escaping.

2. A pouch for use as in claim 1, comprising
an opening and periphery adhesive region for coupling with a stoma, and
a region below said opening and periphery adhesive region of increased length over pouches of the prior art,
whereby said pouch is of adequate length to use at least one times after the bottom part is removed.

3. The method of claim 1, comprising the steps of:
encompassing a cut edge of a pouch with a second pouch or thermoplastic sheet, and
welding together overlapping portions of said pouch and said second pouch or thermoplastic sheet by heat-sealing, and
heat-sealing any open edges of said pouch and said second pouch or thermoplastic sheet,
whereby said cut edge is completely sealed.

4. A second pouch for use as in claim 2, **characterized in that**
at least two edges of said second pouch are closed by means of either heat-sealing or center folding.

5. A pouch and a second pouch for lengthening for use as in claim 1, comprising
an opening and periphery adhesive region for coupling with a stoma in said pouch, and
a region printed with ink or other suitable media in the interior of at least one side of said pouch of the same width of said pouch and of an adequate length below the said periphery adhesive region, or
a thin film of non-thermoplastic film, such as polyester, of the same interior width of said pouch inserted between the sides of said pouch,
whereby said pouch may be lengthened by affixing said second pouch without fusing interior walls.

6. An impulse heat sealer comprising
two facing flexble plastic frames of a substantial length wherein aluminum bars are mounted, and
said aluminum bars to one of which a heating element attaches and to the other of which silicon rubber attaches, and
said plastic frames coupled together with one end for pivoting as fulcrum, and handles on the other ends of said plastic frames as leverage, and
a power circuit including a transformer and a switch mounted out of said plastic frames,
whereby said impulse sealer is lightweight and washable and squeezing pressure is provided to said frames resulting in concentrating pressure on said heating element and said silicon rubber when said frames are held with hands.

7. An impulse heat sealer as in claim 6, **characterized in that**
a lock or a clamp is mounted in the end of the plastic frames of said impulse heat sealer with handles or without the handles.

8. An impulse heat sealer as in claim 7, **characterized in that**
varied pressures may be applied through a lock or a clamp mounted in said impulse heat sealer to a pouch pinched between the frames of said impulse heat sealer to obviate means inside said pouch.

9. An impulse heat sealer as in claim 6, **characterized in that**
a heating wire of said impulse heat sealer coated with an adhesive tape of tetrafluoroethylene exhibits a convex cross-sectional shape.
